# EUROPEAN PATENT SPECIFICATION

(11) **EP 3 037 123 B1**
(45) Date of publication and mention of the grant of the patent: **14.08.2019**
(21) Application number: 15202820.5
(22) Date of filing: 11.10.2011
(51) Int. Cl.: A61M 29/00, A61M 25/10, A61M 31/00, A61J 15/00, A61B 17/34, A61B 17/00, A61M 25/06, A61M 25/00

(54) **A BALLOON CATHETER SYSTEM AND A METHOD OF ASSEMBLING THE BALLOON CATHETER SYSTEM**
BALLONKATHETERSYSTEM UND VERFAHREN ZUR MONTAGE DES BALLONKATHETERSYSTEMS
SYSTÈME DE CATHÉTER À BALLONNET ET PROCÉDÉ D'ASSEMBLAGE DUDIT SYSTÈME

(30) Priority: 15.10.2010 US 905522
(43) Date of publication of application: 29.06.2016
(62) Divisional of application: 11184570.7
(73) Proprietor: Nath, Iyunni Venkata Sesha Sayi, Seminole, FL 33777 (US)
(72) Inventor: Nath, Iyunni Venkata Sesha Sayi, Seminole, FL 33777 (US)
(74) Representative: Kowal, Elzbieta

(56) References cited:
- EP-A1- 0 266 469
- EP-A2- 1 293 228
- WO-A1-2006/087512
- WO-A1-2010/108678
- WO-A2-03/077982
- WO-A2-2005/112801
- US-A- 4 698 056
- US-A- 6 093 173
- US-B1- 6 336 914

## Description

### Field of the Invention

The field relates to a catheter system with detachable dilator for insertion into a patient using an endoscopic percutaneous insertion method or minimally invasive procedures.

### Background

Document US2009/216186A (the "Nath application") discloses a feeding tube. This state of the art does not disclose a catheter system according to claim No 1 with a detachable dilator for use in a percutaneous, minimally invasive procedure that utilizes a detachable dilator. Also, the existing devices are not capable of being used effectively for procedures as varied as gastric feeding, gastric decompression, jejunal feeding, cystostomy, nephrostomy, weight loss and colostomy.

WO 2006/087512 A1 discloses a medico-surgical apparatus of kind comprising a tube with an inflatable sealing cuff, tubular steam arranged in the tube. The tube is assembled on the dilator with dilator extending along the bore of the tube and projecting from its patient end to provide a tapering leading end to the apparatus. The dilator is arranged such that it can be withdrawn rearwardly through the bore of the tube. The dilator has a flexible collar extending rearwadly to overlap the forward end of the tube. The assembly of dilator and tube is inserted to the trachea along a quidewire. The rear end of the quidewire is threaded into the bore at the forward end of the assembly of the dilator and the tube and this assembly is pushed along the quidewire. The collar everting forwardly when the dilator is pulled rearwardly through the tube as to leave the tube in position to permit ventilation in patient's neck. Once the dilator has been removed and the tube is correctly positioned, the sealing cuff is inflated to seal the outside of the tube with the inside of the trachea, in the usual way.

US 6 093 173 A relates to releasable interlock assemblies used in medical devices such as for example catheter introducer assemblies. The invention describes an improvement of a rotatably engaging axial coupling connection between a dilator hub and an introducer hub of catheter introducer. The catheter introducer assembly comprises a dilator portion with dilator hub and a dilator tube with a conical tapered distal end. The dilator hub and the introducer hub are mechanically interlocked.

### Summary of the invention

To solve the above - mentioned problems posed by the prior art, the object of the invention is to provide catheter system with detachable dilator according to nondependent claim No 1
Further exemplary embodiments are evident from the dependent claims.

In the following description, embodiments are described which may not directly relate to the invention, but which may be useful in connection with the invention.

The examples of the invention comprise a catheter system with detachable dilator for use in a wide variety of procedures including, without lone or more of the following procedures: gastric feeding, gastric decompression, jejunal feeding, cystostomy, bladder decompression, colonoscopy, nephrostomy and weight loss. In one example, a sing a balloon device may be used for all of the following indications for use: gastric feeding, gastric decompression, cystostomy, bladder decompression, nephrostomy and colostomy. In another example, a double balloon catheter may be used for one or more of these and for weight loss. In yet another example, a device including a plurality of balloons may be used for one or more of the following: gastric feeding, gastric decompression, jejunal feeding and weight loss.

For example, a catheter includes a balloon inflatable using a fluid, such as water or saline which is capable of acting to retain the catheter in a cavity or lumen, such as the stomach, bladder or colon, for example. When inflated, the balloon is pulled against the wall of the lumen and seals the lumen. The catheter may be inserted under observation using invasive techniques, such as an endoscope. The procedure is modified from the process disclosed in the Nath Application. Instead, a detachable dilator is arranged on the end of the catheter such that an introducer needle, shaft or rod extends through the detachable dilator. The dilator may be detached and retrieved using a snare, which may be used to remove the dilator through an endoscopic passage or other minimally invasive method. Alternatively, the detachable dilator may be made of a material that is capable of being bioabsorbed or may be capable of being detached from the catheter and passing without adverse consequences. For example, certain starch-based materials and gels are capable of being bioabsorbed and/or passing without adverse consequences. Also, it is believed, without being limiting in any way, that a silicone rubber dilator would be capable of passing through the digestive tract without adverse consequences. Other materials for the dilator tip may be a PEEK or a polyethylene, such as high density polyethylene. PEEK is a polyether ether ketone, which may be colorless, and is a known biocompatible engineering thermoplastic material.

The catheter system with detachable dilator of the present invention provides advantages over any known method for inserting a comparatively large catheter, which may be any type of tube, into a cavity or lumen in a patient, which may be a human patient or a non-human patient, such as an animal. Insertion of the catheter is greatly simplified using the detachable dilator. A guide wire, preferably a soft tip guide wire, is inserted in a location where catheterization is desired, using a trochar or other device as is known in the art. The end of the guide wire opposite of the end inserted into the patient is inserted into an annular shaft that extends through the catheter and at least a portion of the dilator. The annular shaft, dilator and catheter tube may be pre-assembled. The assembly may be guided along the guide wire and into the patient, with or without lubrication of the catheter.

For example, the annular shaft may be an introducer needle, which may be made of any stiff biocompatible material, such as a metal. In one example, the metal is stainless steel. The introducer needle is used only during insertion and is removed after the catheter is properly positioned within the patient.

The dilator follows the guidewire into the patient widening the entrance by its transition from about the same diameter as the guide wire or the annular shaft to a diameter about the same diameter or larger than the outer diameter of the catheter. For example, the shape of the nose of the dilator may be a cone, truncated cone or paraboloid. A substantially conical outer surface of a dilator nose may be used, for example. Alternatively, the outer surface of the dilator nose may have a substantially parabolic shape. One advantage of the use of a detachable dilator is that the dilator may have an integrated sheath protecting a balloon of a balloon catheter during insertion of the balloon catheter. Another advantage of a detachable dilator is that the dilator is not withdrawn through the catheter, itself. Therefore, the dilator may have an outer diameter larger than the inner diameter of the tubular device being inserted into a patient. However, unlike external dilators, the catheter need not be inserted through the dilator. Instead, the nose of the dilator may be sized to be the same external diameter as the external diameter of the catheter, because the dilator is dislodged from the catheter after the catheter is properly positioned in the patient. Surprisingly, the detachable dilator reduces the time to perform an insertion procedure while avoiding, or at least reducing, leakage from the cavity around the point of insertion during an insertion procedure.

Surprisingly, a colostomy procedure may use a balloon catheter with a detachable dilator to quickly and efficiently decompress, cleanse and treat a patient that would otherwise be faced with a colostomy. The colostomy uses the minimally invasive surgical techniques similar to a gastrostomy or cystostomy to introduce a balloon catheter in the colon. The balloon catheter may be used for decompression and drainage by connecting the catheter with a colostomy bag. In addition, access to the colon is provided using the catheter for treating an infection or the like. It is surprising and unexpected that essentially the same procedure may be used in not only colostomy but also for nephrostomy, cystostomy, gastrostomy and weight loss. The additional steps required for jejunostomy are the same as required for any jejunostomy procedure. The insertion procedure has fewer complications than known insertion procedures, requires fewer devices to be stocked and improves proficiency by providing a device capable of being used in a plurality of "ostomy" procedures.

A double balloon catheter may be inserted into a patient's stomach in a gastrostomy procedure and may be used for weight loss. By inflating the two balloons, hunger is alleviated, helping the patient to lose weight and to gradually reduce the size of the stomach, while avoiding any blockage of nutrition and hydration. In one example, supplemental nutrition and/or hydration may be provided by gastric feeding, as prescribed by a physician. The dumbbell-shaped double balloon provides a channel for passage of nutrition and hydration through the stomach, while occupying space in the stomach, alleviating discomfort from an empty feeling in the stomach.

In the embodiment of the invention , a catheter for insertion into a patient comprises a balloon catheter, an annular shaft and a detachable dilator. The balloon catheter may be comprised of a tube having a first end, a second end opposite of the first end, a central bore fluidically coupling an inlet at the first end of the tube with an outlet at the second end of the tube and a lumen fluidically coupling a connector disposed adjacent the first end of the tube with a balloon disposed adjacent to the second end of the tube, the balloon being formed by a tubular film sealed at opposite ends of the tubular film to an external surface of the tube. The lumen has a lumen outlet such that fluid injected into the connector and through the lumen exits through the external surface of the tube at the outlet and into an expandable cavity between the balloon, which is made of a flexible, resilient material, and the external surface of the tube. The balloon is capable of being expanded by injecting fluid into the expandable cavity through the lumen.

The detachable dilator may be coupled to the outlet of the tube, when assembled, such that the dilator is held in place during insertion of the tube. After the tube is inserted, the dilator is detached from the tube. The dilator may comprise a sheath portion and a nose, the nose having an external surface that transitions from a first external diameter at a nose tip to a second external diameter at a portion of the nose adjacent to the outlet of tube, when the dilator is coupled to the outlet of the tube. An internal surface of the nose provides the channel through which the annular shaft may be inserted. The sheath portion of the dilator may be formed of a flexible, resilient material, and may be formed in a shape such that it closes in on itself, when the dilator is detached from the tube. The entire dilator may be made of a digestible material and/or bioabsorbable material.

The annular shaft may extend entirely through the nose or may extend through only a portion of the nose of the dilator. For example, the annular shaft may butt up against a portion of the nose of the dilator at a constriction formed within the inner surface of the nose, and the dilator may be detachable from the tube using only the annular shaft, such as by pushing the annular shaft to force the dilator off of the end of the catheter tube. In one example, the annular shaft is attached to a control mechanism, allowing the annular shaft, which extends only partially through the nose, to be used to push the detachable dilator off of the catheter tube, after insertion of the catheter tube into the patient, when the control mechanism is activated. The annular shaft extends through the central bore of the tube and is capable of being removed from the central bore of the tube, after the tube is inserted into the patient, by pulling on an extraction portion of the annular shaft. The extraction portion may extend from the inlet of the tube or may be attached to an extractor that extends from the inlet of the tube. The annular shaft may be made of a stainless steel, for example. In one example, the annular shaft has a pointed tip extending from the nose of the dilator, when the dilator is coupled to the outlet of the tube.

In the embodiment of the invention the catheter system may comprise a separate detachment mechanism in addition to the catheter tube, annular shaft and dilator. For example, the detachment mechanism may comprise a tubular annulus having an inner bore diameter fitting over the outer diameter of the annular shaft and a distal end capable of butting up against the nose of the dilator. By pushing the tubular annulus the detachment mechanism is capable of detaching the dilator from the tube of the catheter, for example. The detachment mechanism may include a control mechanism at a proximal end of the tubular annulus of the detachment mechanism and may include a locking mechanism, which positively locks the tubular annulus until the locking mechanism is unlocked, preventing the distal end of the tubular annulus from pushing the dilator. For example, the locking mechanism may prevent the tubular annulus from being rotated about the longitudinal axis of the tubular annulus. If rotated, about its axis, a guide may be aligned to engage channel allowing the tubular annulus to engage the nose of the dilator, pushing the dilator from the tube of the catheter.

A method of assembling the catheter system according to the invention may comprise inserting the annular shaft and the detachment mechanism through the central bore of the tube and inserting only the annular shaft through at least a portion of the channel of the nose, and coupling the dilator to the outlet of the tube such that the sheath portion of the dilator extends over the outlet of the tube and over at least a portion of the balloon. If the sheath portion of the dilator is formed of a flexible, resilient material, which is closes in on itself, then prior to coupling the dilator to the outlet of the tube, the sheath portion may be unfolded and spread to allow insertion of the outlet portion of the tube into the sheath portion of the dilator.

The method of assembling the catheter system may comprise selecting a pre-assembled catheter system, threading a guide wire through the annular shaft, inserting the dilator, the tube and the annular shaft along the guide wire and into a cavity within the patient, until the balloon is within the cavity, and detaching the dilator from the tube. The annular shaft may be extracted from the tube before or after the dilator is detached. The balloon of the catheter is expanded by injecting fluid into the expandable cavity between the balloon and the external surface of the tube. A retractable net may be inserted into the cavity of the patient to capture the dilator. Then the net and the dilator may be removed from the cavity of the patient, such as through a trochar or endoscope, used for minimally invasive surgical procedures. A control mechanism may be used to control the detachment of the dilator and the control steps may include unlocking the control mechanism, withdrawing the control mechanism beyond a detent in the control mechanism, and rotating the control mechanism about an axis of the control mechanism, prior to pushing the dilator away from the outlet of the tube, using the tubular annulus or the annular shaft or both as a push rod, for example.

### Brief Description of the Drawings

Figures IA - 1C illustrate a detachable dilator as seen (A) externally and (B) in a cross sectional view, while the sheath portion is spread for insertion of the catheter tube, and (C) in a cross sectional view with the sheath portion folded in on itself.
Figure 2 illustrates an example of the use of a detachable dilator, shown in cross section, fitted with a needle introducer and a balloon catheter.
Figure 3 illustrates another example of the use of a detachable dilator, shown in cross section, fitted with a needle introducer, a balloon catheter, in partial cross section, and an example of a detachment mechanism.
Figure 4 illustrates a perspective view of an example of the detachment mechanism's head.
Figure 5 illustrates a perspective view of an example of the detachment mechanism 's handle.
Figure 6 illustrates a partially exploded view of the detachment mechanism and a needle introducer. Figure 6 shows a double balloon catheter. Figure 7 shows a balloon.

### Detailed Description

The examples described and the drawings rendered are illustrative and are not to be read as limiting the scope of the invention as it is defined by the appended claims.

The method of insertion of a balloon catheter using a detachable dilator is similar to the procedure used in the Nath Application. However, instead of withdrawing the needle with a flexible sheath through the large channel in the feeding tube of the Nath Application, only the needle introducer is withdrawn through the catheter. The dilator is detached from the catheter after insertion of the catheter. In one example, the dilator is made of a biocompatible and/or digestible polymer. For example, a polytetrafluoroethylene may be used for the dilator and provides exceptional biocompatibility together with advantageous tribology. However, other polymer materials may be used, such as polyvinylchloride, polyethylene, polypropylene, polyethylene terephthalate, other polyesters, polymethylmethacrylate, polycarbonates, polyamides, polyacetals, polysulfones and co-polymers, such as tetrafluoroethylene and hexafluoropropylene and polyurethane block co-polymers.

In one example, the dilator is withdrawn using minimally invasive techniques. For example, a snare, such as one of the net-like snares capable of being used with an endoscope, is used to capture and remove the dilator. For example, a Roth Net foreign body standard snare may be used.¹
¹ROTH NET^{III} is a trademark of US Endoscopy

In other examples, the dilator is not withdrawn but is made of a bioabsorbable or digestible material or a material that passes harmlessly through the digestive tract. For example, a dilator may be made of a polylactide, which exhibits high tensile strength and rigidity, but hydrolyzing breaks the polylactide down to lactic acid, which digestible or passes harmlessly through the digestive tract. Other bioabsorbable materials include poly(trimethyl carbonate), poly(carbonate) and starch-based polymers and gels, which may provide sufficient rigidity to serve as a dilator.

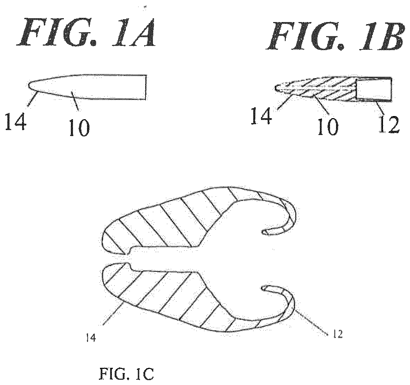

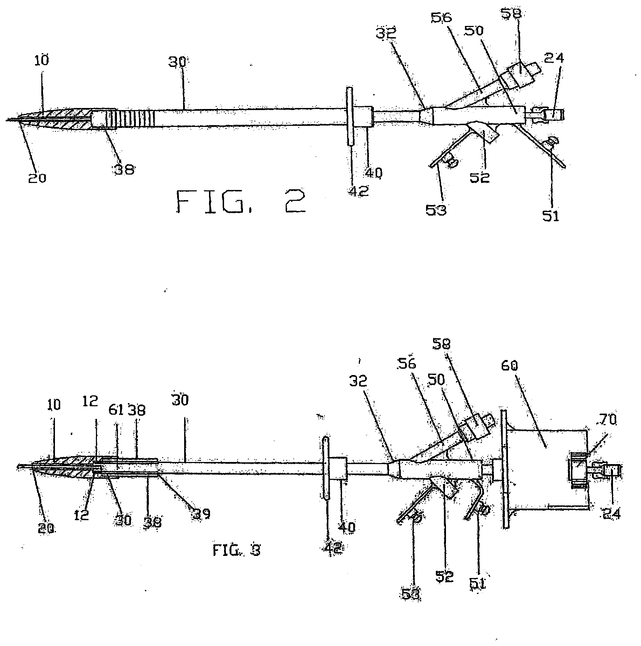

**Figure 1A** illustrates a dilator **10,** having an asymmetric nose 14 and an integrally formed protective sheath 12, as illustrated in the cross sectional view of **Figure 1B****. In** **Figure 1C****,** a resilient, flexible material, such as PEEK or silicone rubber, is used to foul' the sheath portion of the dilator, and the sheath portion is capable of returning to a shape folded in on itself. This may make it possible for the dilator to pass through the digestive tract with no harm to the patient, for example, and reduces the length of the dilator, if it is to be captured by a net, for example. **Figure** 2 illustrates use of the detachable dilator **10** with a needle introducer 20 and a tri-funnel balloon catheter 30. The needle introducer is inserted through a channel defined by the inner diameter of the annular dilator 10. In one example, the needle introducer 20 and dilator 10 are sized, such that the needle introducer may be withdrawn from the dilator 10 and the catheter 30 by pulling the protector 24 while pushing on the catheter 30 to keep the catheter 30 stationary. In this way, both the needle introducer 20 and a guide wire inserted through the needle introducer may be withdrawn from the catheter 30. The dilator 10 is then free to be detached from the catheter 30. In one example, an integrally formed sheath 12 is thin, such as less than 1 millimeter in thickness, and non-rigid, and the dilator 10 dislodges from the catheter 30 as soon as the needle introducer 10 is removed. Alternatively, the dilator 10 may be dislodged from the catheter 30 by expanding the balloon **38,** which is attached at the end of the catheter 30 by adhesive bonds 39 on each end of the balloon, for example. A balloon lumen 56 may be coextruded such that fluid inserted by syringe at the luer lock **58** inflates the balloon with a liquid, such as water or saline. A plurality of balloons may be provided by having additional coextruded balloon lumen, for example. The sheath 12 may protect each of the plurality of balloons, a single balloon or a portion of a single balloon. **Figure** 2 illustrates a sheath protecting a leading edge of a balloon **38,** for example.

An external bolster 40 provides a flange 42 that may be slid downward, once the balloon 38 is inflated and the catheter 30 is in place. In the example of **Figures** 2 and 3, a tri-funnel connector 50 is attached to the tube of the catheter by a transition region 32. The catheter has a main feeding channel, which may be closed by a flexibly attached plug 51 and a secondary drainage port **52,** which may be connected to a drainage bag. The main channel may be flushed with sterile water or saline to keep the catheter free of obstructions and clots. A second plug 53 may be used to close the drainage port 52, when it is not being used. A luer lock 58 seals the balloon inflation lumen **56,** which may be used to fill or drain the balloon 38 using a syringe.

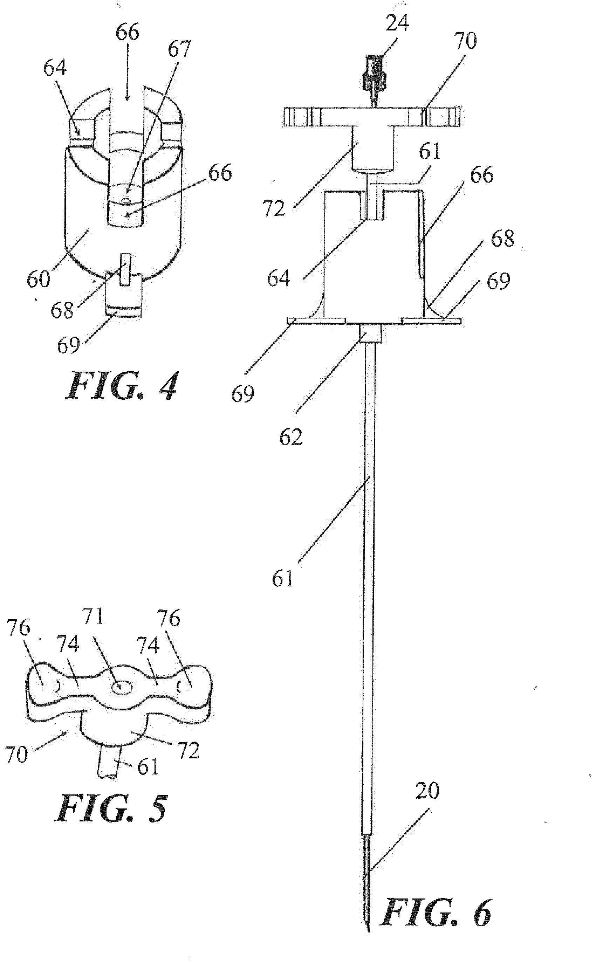

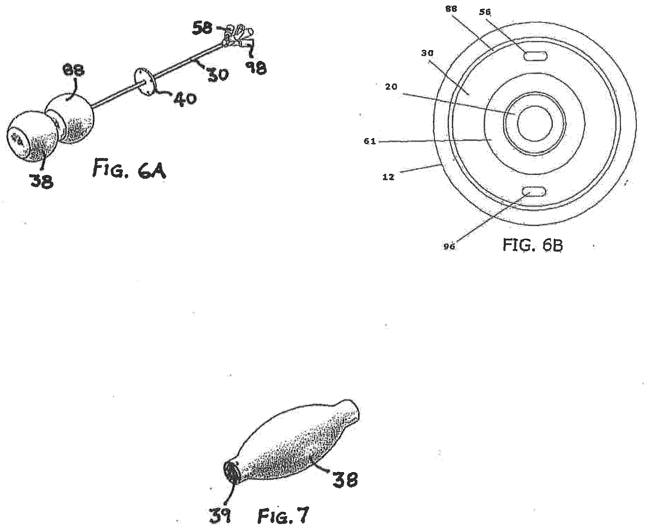

**Figure** 3 illustrates the use of the detachment mechanism 60, 70, which includes a head 60, a handle 70 and a hollow push rod 61, which has a channel through which the needle introducer 20 extends, when preassembled prior to insertion, for example. As illustrated in **Figure 4****,** the head 60 has finger holds 69 on one end and slots or channels **64, 66** on the opposite end. The slots **64, 66** are dimensioned to accommodate the narrow portions 74 of the handle 70. A central bore 67 of the head 60 is sized to accommodate the diameter of the push rod **61.** The push rod 61 is coupled to the handle 70 and one end of the push rod 61 extends from one side of the handle 70. The opposite end of the push rod 61 is capable of contacting the dilator 10 to force detachment of the dilator 10 from the catheter 30, when the handle 70 is backed-off, and rotated 90 degrees in the head 60, as illustrated in Figure 6, aligning the narrow portion 74 of the handle 70 with the elongated channels **66.** The user's fingers may compress the finger holds 76 of the handle 70 toward the finger holds **69** of the head **60,** which are reinforced by arcuate supports **68.** The head 60 has a funnel shape surrounding the central bore 67, which provides a centering function when the push rod 61 is inserted into the central bore **67.** A tapered retention plug 62 extends from the tubeward side of the head 60 and is capable of mating with the main channel of the tri-funnel connector 50 as illustrated in **Figure** 3. A slightly tapered plunger barrel 72 extends into the head 60 and acts to guide the handle 70 into the head 60 during compression. The handle 70 includes a central bore 71 that is capable of accommodating the needle introducer 20, as illustrated in **Figure** 6. The protector 24 on the outward end of the needle introducer 20 may be held with one hand, while the other hand is used to plunge the handle into the head, keeping the needle introducer 20 in place while the dilator 10 is detached from the catheter 30. Alternatively, the needle introducer may be withdrawn prior to detaching the dilator 10 using the detachment mechanism 60, 70.

In **Figure 6A-6B****,** a double balloon (dumbbell-shaped) catheter is illustrated which may not directly be related to the invention, but which may be useful in connection with the invention. The addition of a second balloon **88,** in addition to the balloon nearest the outlet of the catheter 38, is provided to use in patients where a single balloon is insufficient. One or both of the balloons may have a larger volume than a single balloon device. For example, if a single balloon device has a balloon is filled with 20 cubic centimeters of liquid, and then one or both of the double balloons may be filled with 40 cubic centimeters of liquid. Alternatively, each balloon may be filled with the same volume of liquid, for example 20 cubic centimeters. In one example, each of the balloons in the double balloon device are capable of being filled to a maximum volume of 120 cubic centimeters, and use of the catheter is indicated for obese patients that have failed to control weight through diet and exercise. Each balloon has its own, independent luer lock **58, 98** for expansion and contraction of the balloon volumes in **Figures 6A** - **6B****.** The gap between the two balloons **38, 88** provides a path for food and liquid to pass from the esophagus, through the stomach and to the intestines. A feeding port and drainage port are provided as illustrated in the single balloon catheter of **Figures** 2-3.

A typical balloon **38** is illustrated in **Figure** 7, and the adhesive layer 39 is represented by the cross-hatched region on the end of the balloon.

Additional examples and configurations of the features of the examples shall be understood based on these examples and are intended to be included within the scope of the claims, which are not to be limited by the details of the examples provided.

## Claims

1. A catheter system with detachable dilator for insertion into a patient comprising:
a balloon catheter (30) comprised of a tube having a first end, a second end opposite of the first end, a central bore fluidically coupling an inlet at the first end of the tube with an outlet at the second end of the tube and a lumen fluidically coupling a connector disposed adjacent the first end of the tube with a balloon (38) disposed adjacent to the second end of the tube, the balloon (38) being formed by a tubular film sealed at opposite ends of the tubular film to an external surface of the tube, the lumen having a lumen outlet such that fluid injected into the connector and through the lumen exits through the external surface of the tube at the outlet and into an expandable cavity between the balloon (38), which is made of a flexible, resilient material, and the external surface of the tube, whereby the balloon (38) is capable of being expanded by injecting fluid into the expandable cavity through the lumen;
a detachable dilator (10) adapted for being coupled to the outlet of the tube, such that the dilator (10) is held in place during insertion of the tube, and adapted for being detached from the tube after the tube is inserted and not withdrawn , wherein the dilator (10) comprises a sheath portion (12) and a nose (14), the nose (14) having an external surface that transitions from a first external diameter at a nose tip to a second external diameter at a portion of the nose adjacent to the outlet of tube, when the dilator (10) is coupled to the outlet of the tube, and an internal surface providing a channel through the nose (14), the sheath portion (12) extending from the nose (14) and over the outlet of the tube and over at least a portion of the balloon (38),
an annular shaft (20) adapted for being inserted through the central bore of the tube and through at least a portion of the channel of the nose (14), when the dilator (10) is coupled to the outlet of the tube, and the annular shaft (20) is capable of being removed from the central bore of the tube, after the tube is inserted into the patient, by pulling on an extraction portion (24) of the annular shaft (20) extending from the inlet of the tube; and
a separate detachment mechanism (60, 70), the detachment mechanism comprising a tubular annulus (61) having an inner bore diameter fitting over the annular shaft (20) and a distal end adopted for contacting the dilator (10), whereby the separate detachment mechanism (60, 70) is capable of detaching the dilator (10) from the tube by pushing the tubular annulus , wherein the dilator is not withdrawn through the catheter itself.

2. The catheter system of claim 1, wherein the detachment mechanism (60, 70) includes a control mechanism at a proximal end of the tubular annulus (61) of the detachment mechanism.

3. The catheter system of claim 2 , wherein the control mechanism includes a locking mechanism.

4. The catheter system of claim 2, wherein the control mechanism prevents the distal end from pushing the dilator (10), whereby the dilator (10) is detached from the tube, until the tubular annulus (61) is rotated about a longitudinal axis of the tubular annulus (61).

5. The catheter system of claim 1, wherein the sheath portion (12) of the dilator (10) is formed of a flexible, resilient material, and the sheath portion (12) closes in on itself, when the dilator (10) is detached from the tube.

6. A method of assembling the catheter system of claim 1, comprising:
inserting the annular shaft (20) and the tubular annulus (61) of the separate detachment mechanism (60,70); through the central bore of the tube and inserting only the annular shaft (20) through at least a portion of the channel of the nose (14) of the dilator (10); and
coupling the dilator (10) to the outlet of the tube such that the sheath portion (12) of the dilator extends over the outlet of the tube and over at least a portion of the balloon (38).

7. The method of claim 6 wherein the sheath portion (12) of the dilator (10) is formed of a flexible, resilient material, and the sheath portion (12) closes in on itself, prior to coupling the dilator (10) to the outlet of the tube; and the step of coupling includes unfolding and spreading the sheath portion (12) and inserting the outlet of the tube into the sheath portion (12) of the dilator (10).

## Patentansprüche

1. Kathetersystem mit einem diskonnektierbaren Dilatator zum Einführen in einen Patient, umfassend:
einen Ballonkatheter (30), bestehend aus einem Rohr mit einem ersten Rohrende, einem dem ersten Rohrende gegenüberliegenden zweiten Rohrende, eine den Eingang am ersten Rohrende mit dem Ausgang am zweiten Rohrende fluidisch verbindende zentrale Verbindungsöffnung und ein einen benachbart zu dem ersten Rohrende angeordnete Verbinder mit einem an das zweite Rohrende anliegend angeordneten Ballon (38) fluidisch koppelndes Lumen, wobei der Ballon (38) durch eine rohrförmig ausgebildete an den gegenüberliegenden Folienenden zu der Rohraussenseite abgedichtete Folie gebildet ist, wobei das Lumen einen Ausgang aufweist, derart, dass der in den Verbinder und durch das Lumen inijzierte Fluid durch die Rohraussenseite ausgangsseitig und in einen ausdehnbaren aus einem flexiblen, elastischen Material hergestellten Hohlraum zwischen dem Ballon (38) und der Rohraussenseite entweicht, so dass das Ballon (38) durch Inijzieren des Fluids durch das Lumen in den ausdehnbaren Hohlraum ausdehnbar ist;
einen diskonnektierbaren Dilatator (10), ausgebildet zum Verbinden mit dem Rohrausgang, derart, dass der Dilatator (10) beim Einführen des Rohres ortfest gehalten wird, und zum Diskonnektieren von dem Rohr, nachdem das Rohr eingeführt und nicht entfernt wird, wobei der Dilatator (10) einen Abdeckteil (12) und eine Nase (14) enthält, wobei die Nase (14) eine Aussenfläche aufweist, die von dem ersten Aussendurchmesser bei dem Nasenende zum zweiten Aussendurchmesser bei dem am Rohrausgang anliegenden Nasenteil übergeht, wenn der Dilatator (10) mit dem Rohrausgang verbunden ist, und durch die Aussenfläche ein durch die Nase (14), den aus der Nase (14) herausragenden Abdeckteil (12) und oberhalb des Rohrausgangs und mindestens einen Teil des Ballons (38) verlaufender Kanal bereitgestellt wird, einen ringförmigen Schaft (20), ausgebildet zum Einführen durch die zentrale Rohöffnung und durch mindestens einen Teil des durch die Nase (14) verlaufenden Kanals, wenn der Dilatator (10) mit dem Rohrausgang gekoppelt ist, und der ringförmige Schaft (20) aus der zentralen Rohöffnung entfernt werden kann, nachdem das Rohr in den Patient eingeführt wird, indem der sich vom Rohreingang erstreckende Extraktionsteil (24) des ringförmigen Schafts (20) gezogen wird; und
eine separate Diskonnektionseinrichtung (60, 70), wobei die Diskonnektionseinrichtung einen rohrförmigen Ring (61) umfasst, dessen lichte Weite an den ringförmigen Schaft (20) angepasst ist und dessen distale Ende zum Kontaktieren mit dem Dilatator (10) ausgebildet ist, so dass die separate Diskonnektionseinrichtung (60, 70) dazu geeignet ist, den Dilatator (10) von dem Rohr zu diskonnektieren, indem der rohrförmige Ring (61) gedrückt wird, wobei der Dilatator durch den Katheter selbst nicht zurückgezogen wird.

2. Kathetersystem nach Anspruch 1, wobei die Diskonnektionseinrichtung (60, 70) eine Steuerungseinheit am proximalen Ende des rohrförmigen Rings (61) der Diskonnektionseinrichtung umfasst.

3. Kathetersystem nach Anspruch 2, wobei die Steuerungseinheit eine Blockiereinrichtung umfasst.

4. Kathetersystem nach Anspruch 2, wobei das distale Ende durch die Steuerungseinheit gegen dem Drücken des Dilatators (10) gesichert ist, wobei der Dilatator (10) von dem Rohr diskonnektiert wird, wenn der rohrförmige Ring (61) um die Längsachse des rohrförmigen Rings (61) gedreht wird.

5. Kathetersystem nach Anspruch 1, wobei der Abdeckteil (12) des Dilatators (10) aus einem flexiblen, elastischen Material gebildet ist und der Abdeckteil (12) sich selbstständig schliesst, wenn der Dilatator (10) von dem Rohr diskonnektiert wird.

6. Verfahren zum Zusammenbau des Kathetersystems nach Anspruch 1, umfassend:
Einführen des ringförmigen Schafts (20) und des rohrförmigen Rings (61) der separaten Diskonnektionseinrichtung (60, 70) durch die zentrale Rohröffnung und Einführen nur des ringförmigen Schafts (20) durch mindestens einen Teil des durch die Nase (14) des Dilatators (10) verlaufenden Kanals; und Koppeln des Dilatators (10) mit dem Rohrausgang, derart, dass sich der Abdeckteil (12) des Dilatators am Rohrausgang und mindestens am Teil des Ballons (38) erstreckt.

7. Verfahren nach Anspruch 6, wobei der Abdeckteil (12) des Dilatators (10) aus einem flexiblen, elastischen Material gebildet wird und sich der Abdeckteil (12) selbststätig schliesst, bevor der Dilatator (10) mit dem Rohrausgang verbunden wird; und der Kopplungsschritt das Entfalten und das Ausdehnen des Abdeckteils (12) sowie das Einführen des Rohrausgangs in den Abdeckteil (12) des Dilatators (10) umfasst.

## Revendications

1. Système de cathéter avec dilatateur separable pour insertion dans un patient, comprenant:
un cathéter à ballont (30) constitué d'un tube ayant une première extrémité, une deuxième extrémité opposée à la première extrémité, un ouverture central couplant de manière fluidique une entrée située à la première extrémité du tube avec une sortie située à la deuxième extrémité du tube et une lumière couplant de manière fluidique un connecteur disposé de manière adjacente à la première extrémité du tube avec un ballon (38) disposé de manière adjacente à la deuxième extrémité du tube, le ballon (38) étant formé par un film tubulaire scellé aux extrémités opposées du film tubulaire à une surface externe du tube, la lumière ayant une sortie de lumière telle que le fluide injecté dans le connecteur et à travers la lumière sort par la surface externe du tube à la sortie et dans une cavité dilatable située entre le ballon (38), qui est fait d'un matériau souple et élastique, et la surface externe du tube, par quoi le ballon (38) pouvant être dilaté par injection de fluide dans la cavité dilatable à travers la lumière;
un dilatateur separable (10) adapté pour être couplé à la sortie du tube, de sorte que le dilatateur (10) est maintenu en place pendant l'insertion du tube et adapté pour être separé du tube après l'insertion du tube et ne pas être retiré, dans lequel le dilatateur (10) comprend une partie de gaine (12) et un nez (14), le nez (14) ayant une surface externe qui passe d'un premier diamètre externe au bout d'un nez à un deuxième diamètre externe au niveau d'une partie du nez adjacent à la sortie du tube, lorsque le dilatateur (10) est couplé à la sortie du tube, et une surface interne fournissant un canal à travers le nez (14), la partie de gaine (12) s'étendant depuis le nez (14) et au-dessus de la sortie du tube et sur au moins une partie du ballon (38),
un arbre annulaire (20) adapté pour être inséré à travers l'ouverture central du tube et à travers au moins une partie du canal du nez (14), lorsque le dilatateur (10) est couplé à la sortie du tube, et l'arbre annulaire (20) peut être retirée de l'ouverture central du tube, après l'insertion du tube dans le patient, en tirant sur une partie d'extraction (24) de l'arbre annulaire (20) s'étendant à partir de l'entrée du tube; et
un mécanisme de détachement séparé (60, 70), le mécanisme de détachement comprenant un anneau tubulaire (61) ayant un diamètre intérieur d'ouverture s'adaptant sur l'arbre annulaire (20) et une extrémité éloigné adoptée pour entrer en contact avec le dilatateur (10), le mécanisme de détachement séparé (60, 70) est capable de détacher le dilatateur (10) du tube en poussant l'anneau tubulaire, où le dilatateur n'étant pas retiré à travers le cathéter lui-même.

2. Système de cathéter selon la revendication 1, dans lequel le mécanisme de détachement (60, 70) comprend un mécanisme de commande à une extrémité proche de l'anneau tubulaire (61) du mécanisme de détachement.

3. Système de cathéter selon la revendication 2, dans lequel le mécanisme de commande comprend un mécanisme de verrouillage.

4. Système de cathéter selon la revendication 2, dans lequel le mécanisme de contrôle empêche l'extrémité éloigné de pousser le dilatateur (10), le dilatateur (10) est détaché du tube, jusqu'à ce que l'anneau tubulaire (61) est tourné autour d'un axe longitudinal de l'anneau tubulaire (61).

5. Système de cathéter selon la revendication 1, dans lequel la partie de gaine (12) du dilatateur (10) est formé d'un matériau élastique et souple, et la partie de gaine (12) se ferme sur elle-même, lorsque le dilatateur (10) est détaché du tube.

6. Procédé d'assemblage du système de cathéter selon la revendication 1, comprenant:
insérer l'arbre annulaire (20) et l'anneau tubulaire (61) du mécanisme de détachement séparé (60, 70); à travers l'ouverture central du tube et insérer uniquement l'arbre annulaire (20) à travers au moins une partie du canal du nez (14) du dilatateur (10); et coupler le dilatateur (10) à la sortie du tube de telle sorte que la partie de gaine (12) du dilatateur s'étend au-dessus la sortie du tube et au-dessus au moins une partie du ballon (38).

7. Procédé selon la revendication 6, dans lequel la portion de gaine (12) du dilatateur (10) est formée d'un matériau souple et élastique, et la partie de gaine (12) se ferme sur elle-même avant l'accouplement le dilatateur (10) à la sortie du tube; et l'étape de couplage comprend le dépliage et étaler la partie de gaine (12) et insérer la sortie du tube dans la partie de gaine (12) du dilatateur (10).
